# EUROPEAN PATENT SPECIFICATION

(11) **EP 3 206 570 B1**
(45) Date of publication and mention of the grant of the patent: **25.06.2025**
(21) Application number: 15850944.8
(22) Date of filing: 13.10.2015
(51) Int. Cl.: A61B 5/00, G06F 1/16

(54) **SYSTEMS, DEVICES, AND METHODS FOR DYNAMIC CONTROL**
SYSTEME, VORRICHTUNGEN UND VERFAHREN ZUR DYNAMISCHEN STEUERUNG
SYSTÈMES, DISPOSITIFS ET PROCÉDÉS DE COMMANDE DYNAMIQUE

(30) Priority: 13.10.2014 US 201462063137 P
(43) Date of publication of application: 23.08.2017
(73) Proprietor: Google LLC, Mountain View, CA 94043 (US)
(72) Inventor: VU, Sonny X., Salem, New Hampshire 94014 (US); GOLNIK, Timothy, Dallas, Texas 75243 (US); DIAMOND, Steven, San Francisco, California 94110 (US)
(74) Representative: Thorniley, Peter
(86) International application number: PCT/US2015/055260
(87) International publication number: WO 2016/061056

(56) References cited:
- US-A1- 2005 121 504
- US-A1- 2007 033 069
- US-A1- 2008 103 794
- US-A1- 2008 104 012
- US-A1- 2010 331 147
- US-A1- 2014 088 922
- US-A1- 2014 088 922
- US-A1- 2014 122 537
- US-A1- 2014 135 592
- US-B1- 6 689 057

## Description

### Background

Embodiments described herein relate generally to systems, devices, and methods for dynamic control using user input. The ubiquity of cloud-based applications running on Smartphones has led to a significant change in how we provide tactile inputs that trigger responses from our environment. Moreover, devices are increasingly interconnected and in communication with each other, thus a generally tech-savvy user, for example, can have a home environment that interconnects his Smartphone, telephone, personal computer, tablet device, television, a digital video recorder, a Bluetooth speaker, an adaptive thermostat such as that provided by Nest, and/or the like. While some of these devices can be used to control the other(s), the ability to do so is hindered by the need to render this complexity of interaction to the user (for purposes of enabling the user to make a selection) and the resulting user inconvenience. For example, a Smartphone can be used to control most of the above mentioned devices via different device-specific applications, which requires the user to constantly switch between applications. As another example, even if the Smartphone has a iversalremote control" application for the various devices, the application usually includes a complex interface with different controls for each device.

There is hence an unmet need to expand the possible actions a user can take while maintaining simplicity of the interface and input available to the user.

US2014/088922 discloses a device and method for processing fitness information.

### Summary

The following detailed description and the accompanying drawings disclose a number of examples, aspects and embodiments provided for the understanding of the invention. The invention is disclosed in the passages identified as claimed embodiments, and the relative drawings if any, and its scope is defined uniquely by the appended claims.

### Brief Description of the Drawings

FIG. 1 is a schematic illustration of a setup for dynamic control, according to an embodiment.
FIG. 2 is a method of dynamic control, according to an embodiment.
FIGS. 3A-3B are various views of a personal fitness device, according to an exemplary embodiment.
FIGS. 3C-3D are various views of the personal fitness device of FIGS. 3A-3B held in a clasp, according to an exemplary embodiment.
FIGS. 3E-3F are various views of the personal fitness device of FIGS. 3A-3B held in a wrist strap, according to an exemplary embodiment.

### Detailed Description

Systems, devices and methods are described herein that enable a user to exercise dynamic control over a controllable entity, such as a smartphone, appliances, vehicles, and/or the like. Embodiments describe herein provide for real-time, automated determination of the context of the user's inputs, such as based on, for example, the user's activity, location, and/or environment.

There is increasing digital interconnectness provided by everyday devices and/or systems that can constantly monitor and/or manipulate a user's existential experience. In turn, the user can be enabled to manipulate such devices, often remotely, to affect his digital and/or real-world environment. Some examples of such user devices and the corresponding user actions can include, but is not limited to, a Bluetooth headset wirelessly connected to a smartphone that, when a call is incoming, allows a user to take a phone call hands-free; a Smartphone application that allows a user to control a digital television via a household WiFi signal; an elder care device including a button, worn around the neck, and usable to signal an alarm to a healthcare practitioner in times of distress; and/or the like.

Such actions are almost always undertaken by the user in the context of his needs, desires, state of mind, state of body, his environment, etc. In all of these cases, however, the user interface that receives the user input is configurable for singular action, and is context-agnostic. In some cases, the interface can be reprogrammed to perform another action. For example, a product called "bttn" aims to make a particular digital action available to anyone at the push of a physical button. However, the approach employed by bttn is still context-agnostic, and limits the user's ability to affect a wide range of actions using a relatively simplistic interface.

Accordingly, aspects of this disclosure permits a user to affect a wide range of actions with a simple user interface and approach that automatically accounts for the user's digital and/or real-world state, based on the inputs and/or abilities of various interconnected devices/systems associated with the user, to select the action to be performed. For example, in some exemplary embodiments described herein, a button-type device can be configured an alarm generator if the person is indoors (e.g., as indicated by the wireless proximity of a digital television), can hail a cab via a taxicab smartphone application (e.g., Uber) if the person is near a road (e.g., as indicated by a GPS sensor on a wirelessly connected smartphone), and/or initialize a mapping smartphone application if the person is in a car (e.g., as indicated by a wirelessly connected car GPS system). A dial-type device according to aspects disclosed herein can be configured to allow a user to scroll through a playlist on a wirelessly connected smartphone when the user is playing music, to dim room lights on a wirelessly connected light controller when within a detectable range, to increase the volume on a Bluetooth connected speaker nearby, and/or the like.

As used in this specification, the singular forms "a," "an" and "the" include plural referents unless the context clearly dictates otherwise. Thus, for example, the term "a network" is intended to mean a single network or a combination of networks.

A method includes receiving, wirelessly from a first device, at a second device, user input information associated with a user, the first device (100) comprising one or more input sensors or interfaces for receiving input from the user. The method also includes receiving, wirelessly at the second device, additional information associated with the user, the additional information including information from a third device. The method also includes identifying, based on the user input information and the additional information, one or more actions for 3 xecution including traversing a structure of a plurality of actions, the structure selected from the group consisting of a directed graph, an undirected graph, a finite state model, a decision tree, and a flowchart. The method also includes transmitting, from the second device, an indication of the one or more actions to be executed to the third device wirelessly.

In some embodiments, the method also includes receiving, from the first device, at the second device, fitness information associated with the user. The method may also include identifying, based on the user input information and the fitness information, one or more actions.

In some embodiments, a first device (sometimes also referred to as a "personal fitness device") includes one or more input sensors or interfaces for receiving input from a user. In some embodiments, the user input can include binary input, analog input, and/or combinations thereof. In some embodiments, the first device can also include additional fitness sensors for monitoring, tracking, and/or otherwise determining fitness parameters/data associated with a user. The first device can also include one or more storage media for storing the user input and/or the fitness data, and one or more processors for controlling operation of the first device. The first device can also include one or more communication modules for wirelessly communicating and/or otherwise transferring the user input and/or the fitness data, or information associated therewith, such as to a second device, for example. In some embodiments, the transfer of user input information can be done in real-time and/or continuously. In other words, the first device can acquire and transmit the user input in a substantially continuous manner. In some embodiments, the transfer of the fitness information can be done in real-time and/or continuously. In other words, the first device can acquire and transmit the fitness parameters in a continuous manner. In other embodiments, the fitness information can be transferred on a periodic basis, e.g., every few hours, or based on a user initiated syncing operation.

The first device can also include one or more power sources. The one or more power sources of the first device can include, but is not limited to, replaceable batteries such as button cells, an integrated battery, a rechargeable battery (including an inductively-rechargeable battery), capacitors, super-capacitors, and/or the like. In some embodiments, the first device can include a button cell, so as to be operable for several months without requiring replacement. In some embodiments, the first device can include a power switch for powering the first device on and off, while in other embodiments, the first device does not have a power switch that can be manipulated by a user. In some embodiments, the first device can be powered on and off by the second device.

In some embodiments, the user input can be received at the first device in any suitable manner, such as, but not limited to, via spoken commands, via tactile entry (e.g., via a button, a keypad, a touch-sensitive screen/panel), via motion (e.g., moving the first device in a circle, detectable via an accelerometer or gyroscope), via sensing (e.g., via a temperature sensor upon user touch), and combinations thereof. For example, in some embodiments, the user input can be received via prolonged operation of a button (e.g., clicking the button for at least 2 seconds), as well as rotation of the button. Accordingly, the one or more input sensors can include, but are not limited to, one or more of an audio receiver (e.g., a microphone), a button, a keypad, a dial, a touchscreen, electrical sensors, conductance sensors, accelerometers, magnetometers, gyroscopes, capacitive sensors, optical sensors, cameras, global positioning system (GPS) sensors combinations thereof, and/or the like.

The fitness data can be physiological, geospatial/timing, and/or the like, in nature. Examples of physiological data include, but are not limited to, heart and/or pulse rate, blood pressure, muscle electrical potential, nerve electrical potential, temperature, brain waves, motion, measures of activity, number of steps taken, and/or the like. Examples of geospatial and/or timing data include but are not limited to, location, acceleration, pace, distance, altitude, direction, velocity, speed, time elapsed, time left, and/or the like. Accordingly, the one or more fitness sensors can include, but are not limited to, one or more temperature sensors, electrical sensors, conductance sensors, accelerometers, magnetometers, gyroscopes, capacitive sensors, optical sensors, cameras, global positioning system (GPS) sensors, and/or the like.

The one or more communication modules can be implemented in software (e.g. as a communication module stored in the storage media or of the one or more processors) and/or hardware (e.g. as a separate circuit, antenna, speakers, light emitting diodes (LEDs), etc.) to enable any suitable communication protocol. The communication protocol can include, but is not limited to, Bluetooth, low power Bluetooth (BLE), near field communication (NFC), radio frequency (RF), Wi-Fi, and/or the like. In some embodiments, the communication protocol can include audio-based protocols such as using a modem to transmit data using audio frequencies and/or ultrasonic frequencies. In some embodiments, the communication protocol can include light-based optical data transfer, such as a pattern of blinking LEDs or a single blinking LED, for example. In some embodiments, the communication protocol can encompass variation of a magnetic field associated with the first device, such as with an electromagnet of the first device.

The one or more storage media of the first device can be any suitable storage media for storing the user input and/or the fitness data. In some embodiments, the storage media include non-transitory computer-readable media, as described below. In some embodiments, the storage media include non-volatile computer storage media such as flash memory, EEPROM (Electrically Erasable Programmable Memory), FRAM (Ferroelectric Random Access Memory), NVRAM (Non Volatile Random Access Memory), SRAM (Static Random Access Memory), and DRAM (Dynamic Random Access Memory). The one or more processors can be any suitable processing device for controlling operation of the various components of the first device. In some embodiments, one or more modules are implemented on the storage media and/or the processor for controlling operation of the first device.

The second device can be any device including at least one communication module configured for communicating with the first device, using a suitable communication protocol as described above. In some embodiments, the first device 100 and the second device 160 are configured for proximity based data transfer of the fitness data, as briefly discussed below. During operation, the second device is configurable to determine that the first device is in physical proximity. In some embodiments, a communication component/module of the second device can be used to determine proximity. In other embodiments, a communication component/module of the first device can be used to detect proximity, and some other means (such as audio) can be used to trigger a sensor of the first device. In some embodiments, determining physical proximity includes instantaneously detecting the presence of the first device by a sensing component/module of the second device, such as when the first device and the second device are placed in momentary or persistent contact with each other or 'bumped' together, for example. In other embodiments, a sensed component/module of the first device can be used to detect contact between the two devices. In some embodiments, determining physical proximity includes detecting the presence of the first device by the sensing component/module of the second device for a predetermined and/or programmable duration of time. In this manner, the system and method can be configurable to ensure that the first and second devices are likely to remain in proximity before initiating data transfer. In some embodiments, determining physical proximity includes detecting the presence of the first device to be within a predetermined and/or programmable distance of the second device, such as might be inferred by the strength of the signal output from the sensing component/module of the second device, for example. In some embodiments, determining physical proximity includes detecting the presence of the first device to be within a predetermined and/or programmable distance of the second device, such as detecting continued contact, for example as might be measured when a sufficiently conductive portion of device is in close enough proximity with a capacitive touch screen of the second device, or for example if a magnetic element of the first device is in sufficiently close proximity with a magnetometer of the second device. In some embodiments, once the first and second devices are deemed to be in physical proximity, the second device is further configurable to transmit a control signal to the first device to initiate data transmission of the stored fitness parameters via a communication link, and is further configurable to store, transmit, and/or analyze the received data.

In some embodiments, the second device additionally includes an action module configured to dynamically identify an action based on the received user input information, on the received fitness information, or both. In some embodiments, the action module is further configured to identify the action(s) based on additional information such as, but not limited to, data obtained from other modules/components of the second device, data obtained from another device, and/or the like. Illustrative, non-limiting examples of additional information include, but are not limited to, an indication of music being played by the second device, an indication of the geospatial location of the second device, an indication of the second device being located near another device in a household of the user, any previous action identified by the action module, and/or the like.

The action module identifies the action based on the user input information, and additionally based on the additional information and optionally on the fitness information. A few illustrative examples of such actions can include:
The user input information specifies a button click on the first device, and the fitness information specifies an elevated heart rate; the action can include initiating a music player and playing a high beats per minute (BPM) song;
The user input information specifies a clockwise rotation of a dial/button on the first device, and the additional information specifies that a slideshow of photographs is currently being displayed on the second device; the action can include advancing the slideshow to the next photograph;
The user input information specifies a tap on a touchscreen of the first device, and the additional information, provided by a light controller in the user's living room to the second device, specifies that the user is in the living room and that the lights are currently switched off; the action can include switching on the lights; and
The user input information specifies a swipe pattern on a touchscreen of the first device, and the fitness information includes GPS coordinates indicating the user is on a roadway; the action can include turning on a GPS capability of the second device and initializing a mapping application with a destination determined by the swipe pattern.

In some embodiments, there is no action identifiable based on the combination of user input information / fitness information / additional information provided to the action module. In such embodiments, either no action can be taken, or a predetermined default action can be taken.

In this manner, the action module is configured to dynamically identify what action should be taken based on available information, and can take account of the user's personal state, the user's personal surroundings, of the user's usage of the second device and/or any other device, and/or the like. The second device can then act as a dynamically configurable controller that can respond to identical user input differently, depending on other circumstances indicated by the fitness information and/or the additional information; and/or contrastingly, respond to different user input in substantially the same way.

In some embodiments, the one or more actions can be can be defined, updated, and/or manipulated by any suitable entity including, but not limited to, a user associated with the first device, a user associated with the second device, received from another device (not shown), and/or the like. An action can be identified and/or otherwise selected from a plurality of actions that can be supplied in any suitable format permitting traversal by the action module for purposes of identifying the necessary action. As illustrative examples, the plurality of actions can be structured / illustrated as one or more of a directed graph, an undirected graph, a state diagram including a finite number of states, a flowchart, provided via an IFTTT ("If This Then That"), a decision tree, and/or the like. In some embodiments, the plurality of actions is stored at the second device (e.g., in a memory and/or database of the second device), while in another embodiment, the plurality of actions is stored on a remote storage accessible by the second device.

In some embodiments, the action module is configured to execute the identified action. In another embodiment, the action module transmits the identified action, or information associated therewith, to another module of the second device. In yet another embodiment, the action module transmits the identified action, or information associated therewith, to another device, that may or may not be the first device, for execution. For example, when the action specifies that the room lights should be turned on, the action module can be configured to transmit information associated with the identified action to a controller for the room lights, via a wireless connection, for example.

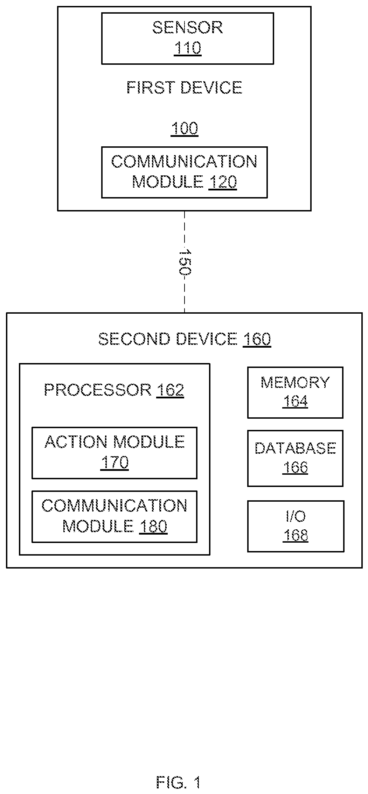

FIG. 1 is a schematic illustration of a wireless setup/system for dynamic control, according to an embodiment. The first device 100 is operable for use by a user for collecting user-specific information, such as user input, fitness-related information, biometric information, and/or the like. In some embodiments, the first device 100 can include a personal fitness device or activity tracker such as, but is not limited to, a pedometer, a physiological monitor such as a heart rate monitor, a respiration monitor, a GPS system (including GPS watches), and/or the like. The first device 100 includes at least a user input sensor 1 10, and a communication module 120. The first device 100 can further include fitness sensors, storage media, and processor(s) (not shown) as described earlier as suitable for collecting, storing, and transmitting the fitness data.

The first device 100 can be in communication with the second device 160 via a communication link 150 as shown in FIG. 1 via a network. The communication link 150 can be any suitable means for wireless communication between the first device 100 and the second device 160, including capacitive, magnetic, optical, acoustic, and/or the like. The communication link 150 can include bidirectional communication between the first device 100 and the second device 160. In some embodiments, any or all communications may be secured (e.g., encrypted) or unsecured, as suitable and as is known in the art.

The second device 160 can include any device/system capable of receiving user input information from the first device 100. In some embodiments, the second device 160 can include a personal computer, a server, a work station, a tablet, a mobile device (such as a Smartphone), a watch, a cloud computing environment, an appliance (e.g., lighting, television, stereo system, and/or the like), an application or a module running on any of these platforms, a controller for any of these platforms, and/or the like.

In some embodiments, the second device 160 is a Smartphone executing a native application, a web application, and/or a cloud application for implementing aspects of the second device 160 disclosed herein. In some embodiments, the first device 100 and the second device 160 are commonly owned. In some embodiments, the first device 100 and the cloud application executing on the second device 160 are commonly owned. In other embodiments, the second device 160 and/or the cloud application executing on the second device are owned by a third party with respect to the first device 100.

The second device includes at least a processor 162 and a memory 164. FIG. 1 also illustrates a database 166, although it will be understood that, in some embodiments, the database 166 and the memory 164 can be a common data store. In some embodiments, the database 166 constitutes one or more databases. Further, in other embodiments (not shown), at least one database can be external to the second device 160. FIG. 1 also illustrates an input/output (I/O) component 168, which can depict one or more input/output interfaces, implemented in software and/or hardware, for other entities to interact directly or indirectly with the second device 160, such as a human user of the second device 160.

The memory 164 and/or the database 166 can independently be, for example, a random access memory (RAM), a memory buffer, a hard drive, a database, an erasable programmable read-only memory (EPROM), an electrically erasable read-only memory (EEPROM), a read-only memory (ROM), Flash memory, and/or so forth. The memory 164 and/or the database 166 can store instructions to cause the processor 162 to execute modules ,processes and/or functions associated with the second device 160.

The processor 162 can be, for example, a general purpose processor, a Field Programmable Gate Array (FPGA), an Application Specific Integrated Circuit (ASIC), a Digital Signal Processor (DSP), and/or the like. The processor 162 can be configured to run and/or execute application processes and/or other modules, processes and/or functions associated with the second device 160 and/or a network associated therewith.

The second device 160 includes an action module 170 for identifying one or more actions based on the user input information and further based on the fitness information and/or the additional information. The second device 160 further includes a communication module 180 for communicating with the first device 100 via the communication link 150 (i.e. for communicating with the communication module 120 of the first device).

The communication module 180 can be configured to facilitate network connectivity for the second device 160. For example, the communication module 140 can include and/or enable a network interface controller (NIC), wireless connection, a wired port, and/or the like. As such, the communication module 180 can establish and/or maintain a communication session with the first device 100. Similarly stated, the communication module 140 can enable the system 100 to send data to and/or receive data from the first device 100, and/or other devices (not shown).

In some embodiments, the processor 162 can include additional modules (not shown). Each module can independently be a hardware module and/or a software module (implemented in hardware, such as the processor 162). In some embodiments, the modules 170, 180 can be operatively coupled to each other.

During operation, a user can engage the first device 100 in any suitable manner to generate a user input signal via the sensor 110, which can be a plurality of input sensors. For example, sensor 110 can encompass a clickable button (singular input) that is also rotatably attached (either directly or indirectly) to an accelerometer to generate a continuous signal (continuous input). The first device 100 can communicate information associated with the user input (i.e., user input information) to the communication module 180 of the second device via a communication module 120 using any suitable protocol, such as, for example, low power Bluetooth, wifi, RF, NFC, or the like. In some embodiments, the user input information is communicated by the first device 100 to the second device 160 substantially in real time and/or in a continuous manner.

In some embodiments, the first device 100 can generate, and/or have stored thereon, fitness data generated by fitness sensors (not shown) of the first device. In some embodiments, information associated with the fitness data (i.e., fitness information) is transmitted in real time to the second device 160, while in other embodiments, the first device 100 stores the fitness information in a storage (not shown) of the first device, and transmits it to the second device 160 at a later time.

In some embodiments, the first device 100 and the second device 160 are further configured to transfer the data therebetween via a communication protocol selected from:
Bluetooth, low power Bluetooth (BLE), near field communication (NFC), radio frequency (RF), Wireless-Fidelity (Wi-Fi), an audio-based protocol, a light-based protocol, a magnetic field-based protocol, an electric-field based protocol, and combinations thereof.

In some embodiments, the action module 170 of the second device 160 is configured to receive the user information and the fitness information, and is further configured to receive additional information, which can be any information other than the user information and the fitness information. The additional information can be sourced from the second device 160, an application/module executing on the second device, another device (not shown), and/or the like. In some embodiments, the action module 170 is further configured to identify, based on any combination of the user input information/fitness information/additional information, one or more actions.

Non-limiting example combinations, in addition to those already discussed, of user information/fitness information/additional information, and the identified action, are listed in Table 1.

**Table 1**

| User Input Information | Fitness Information | Additional Information | Action(s) |
|---|---|---|---|
| Button Click+Counterclockwise Rotation | - | Earphones are plugged in, and music application on the second device is playing a song | Select and play previous song on playlist in music application |
| Touchscreen Click | Acceleration detected | - | Initialize/resume run timer |
| Moving the First Device in a Circle | - | Incoming phone call on the second device | Reject phone call |
| Clockwise Button/Dial Rotation | Lowered heart rate | After 8pm | Dim room lights, lower room temperature |
| Audio Command "**repeat**" | - | Last action was to toggle Ai lane mode | Toggle Airplane Mode |
| Touchscreen Click | - | The user is outdoors (GPS application), and has a lunch appointment in 5 minutes 12 miles away (Calendar application | Initialize an application for seeking a taxicab service (e.g., Uber) |

In a claimed embodiment, the action module 170 and/or the communication module 180 is configured to transmit information associated with the identified action to an entity capable of executing the identified action, such as a third device (not shown).

Some embodiments described herein can relate to a kit including the first device and/or the second device. In some embodiments, the kit can include one or more holders for the first device and/or the second device. As an example, a kit can include the first device 100, and further include one or more accessories for holding the device such as a necklace, a wrist strap, a belt, a clip, a clasp, and/or the like.

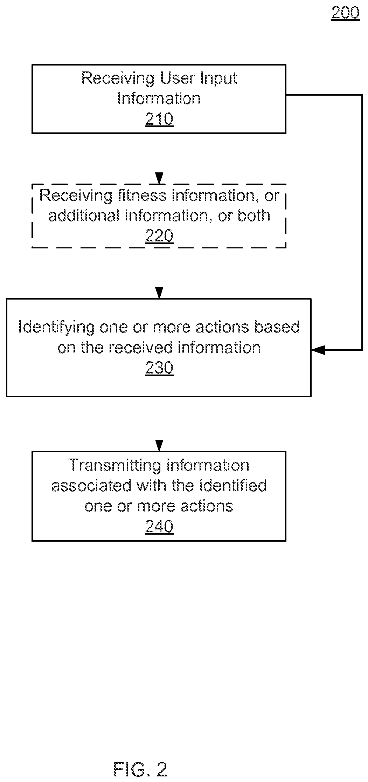

FIG. 2 illustrates a method 200 of dynamic control, according to embodiments. Explained here with reference to FIG. 1, the method 200 can be executed by the second device 160, or any structural/functional variant thereof. At 210, the method 200 includes receiving user input information, such as from the first device 100, for example. At step 220 (as indicated by dotted lines), the method 200 optionally includes receiving fitness information (e.g., from the first device 100), and includes receiving additional information (e.g., from other modules/applications of the second device 160, from yet another device, etc.). At 230, subsequent to or alternative to step 220, the method 200 includes identifying one or more actions based on the received information. The one or more actions is identified based at least on the received user input information and the additional information, and (optionally) on the received fitness information. At 230, the method 200 includes transmitting information associated with the identified one or more actions, such as to, for example, another module/application of the second device 160, another device (not shown), and/or the like. In some embodiments, the transmitted information associated with the identified one or more actions includes an instruction for executing the one or more actions.

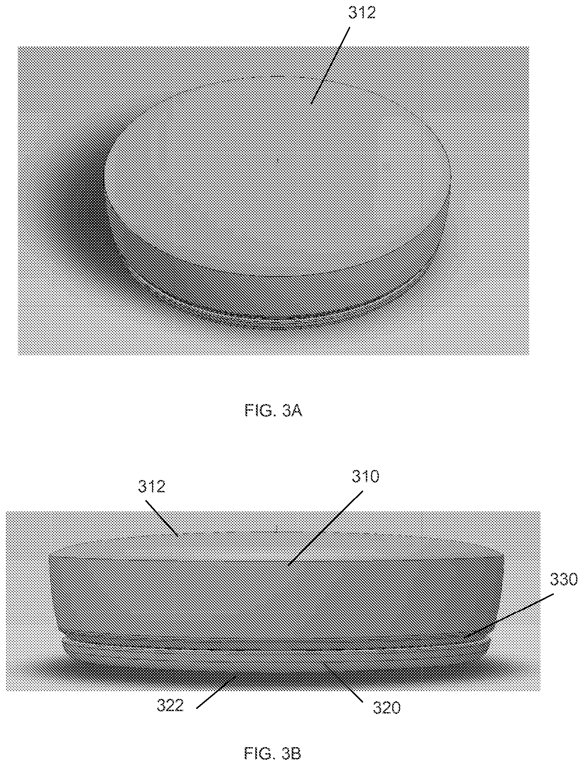

As illustrated in FIGS. 3A-3B, in some embodiments, a personal fitness device 300 (which can be substantially similar to the first device 100) can be designed as a frustum-shaped structure having a first portion 310 and a second portion 320. The first portion 310 can include a first, generally convex surface 312 that can be configured to receive user input, and a ridge 314. For example, the first surface 312 can include a touchscreen. As another example, the first portion 310 can constitute a depressible and/or rotatable (relative to the second portion 320) button configured for receiving user input. In some embodiments (not shown), the first surface 312 can include a plurality of independently controllable light indicators such as, for example, LEDs, as disclosed in the '195 application. In some embodiments (not shown), the first surface 3 12 can include an indentation that permits a user to more easily lodge a finger and rotate the first portion 310 or can rotate the whole device 300. In some embodiments (not shown), the first surface 312 can include a display, such as for displaying date and/or time, for example.

In some embodiments, one or more fitness sensors can be included in the first portion 310 and/or the second portion 320. In some embodiments, a second surface 322 of the second portion 320 can include one or more fitness sensors (e.g., for measuring heart rate) for interfacing with the skin of the user during use.

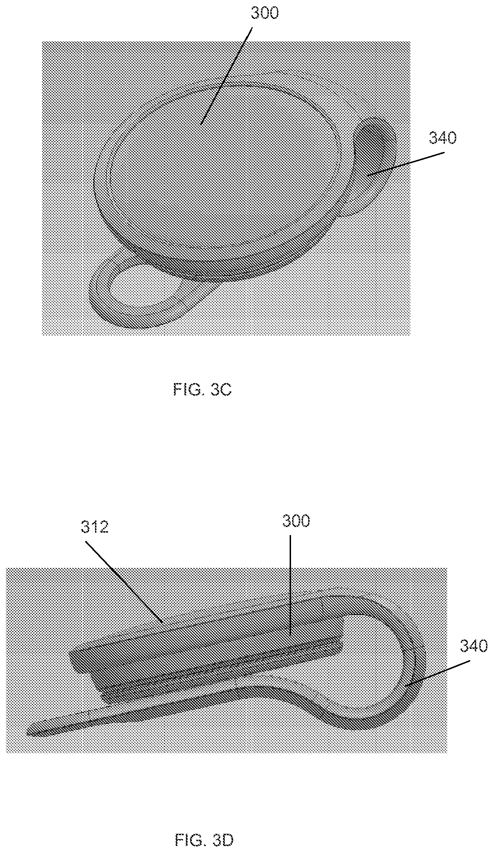

FIGS. 3C, 3D illustrate a perspective and side-view, respectively, of the device 300 releasably held within a clasp 340. In this manner, a user can clip the device 300 onto a garment/other accessory (e.g., a backpack), and still manipulate the first surface 312 to provide user input. In some embodiments (not shown), the clasp 340 can include a rotatable receiving portion for the device 300, such that rotation of the rotatable receiving portion by a user in turn rotates the entire device 300 or the first portion 3 10, thereby providing user input as described earlier.

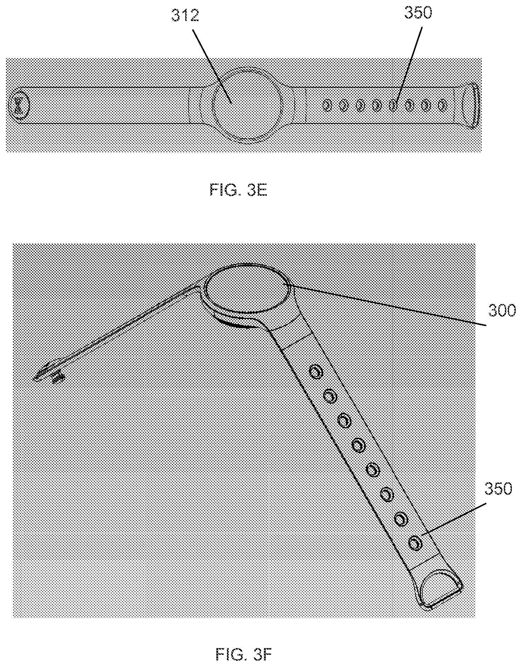

FIGS. 3E, 3F illustrate a top and perspective view, respectively, of the device 300 releasably held within a wrist strap 350. In this manner, a user wear the device 300 on their wrist, manipulate the first surface 312 to provide user input, and be in physical contact with the second surface 322 to provide fitness data via the second surface. In some embodiments (not shown), the wrist strap 350 can include a rotatable receiving portion for the device 300, such that rotation of the rotatable receiving portion by a user in turn rotates the entire device 300 or the first portion 3 12, thereby providing user input as described earlier.

Some embodiments described herein relate to a computer storage product with a non-transitory computer-readable medium (also referred to as a non-transitory processor-readable medium) having instructions or computer code thereon for performing various computer-implemented operations. The computer-readable medium (or processor-readable medium) is non-transitory in the sense that it does not include transitory propagating signals (e.g., a propagating electromagnetic wave carrying information on a transmission medium such as space or a cable). The media and computer code (also referred to herein as code) may be those designed and constructed for the specific purpose or purposes. Examples of non-transitory computer-readable media include, but are not limited to: flash memory, magnetic storage media such as hard disks, optical storage media such as Compact Disc/Digital Video Discs (CD/DVDs), Compact Disc-Read only Memories (CD-ROMs), magneto-optical storage media such as optical disks, carrier wave signal processing modules ,and hardware devices that are specially configured to store and execute program code, such as Application-Specific Integrated Circuits (ASICs), Programmable Logic Devices (PLDs), Read-Only Memory (ROM) and Random-Access Memory (RAM) devices.

Examples of computer code include, but are not limited to, micro-code or microinstructions, machine instructions, such as produced by a compiler, code used to produce a web service, and files containing higher-level instructions that are executed by a computer using an interpreter. For example, embodiments may be implemented using Java, C++, or other programming languages and/or other development tools.

Where methods and/or schematics described above indicate certain events and/or flow patterns occurring in certain order, the ordering of certain events and/or flow patterns may be modified. Additionally certain events may be performed concurrently in parallel processes when possible, as well as performed sequentially.
300. In some embodiments (not shown), the first surface 312 can include a display, such as for displaying date and/or time, for example.

In some embodiments, one or more fitness sensors can be included in the first portion 310 and/or the second portion 320. In some embodiments, a second surface 322 of the second portion 320 can include one or more fitness sensors (e.g., for measuring heart rate) for interfacing with the skin of the user during use.

FIGS. 3C, 3D illustrate a perspective and side-view, respectively, of the device 300 releasably held within a clasp 340. In this manner, a user can clip the device 300 onto a garment/other accessory (e.g., a backpack), and still manipulate the first surface 312 to provide user input. In some embodiments (not shown), the clasp 340 can include a rotatable receiving portion for the device 300, such that rotation of the rotatable receiving portion by a user in turn rotates the entire device 300 or the first portion 310, thereby providing user input as described earlier.

FIGS. 3E, 3F illustrate a top and perspective view, respectively, of the device 300 releasably held within a wrist strap 350. In this manner, a user wear the device 300 on their wrist, manipulate the first surface 312 to provide user input, and be in physical contact with the second surface 322 to provide fitness data via the second surface. In some embodiments (not shown), the wrist strap 350 can include a rotatable receiving portion for the device 300, such that rotation of the rotatable receiving portion by a user in turn rotates the entire device 300 or the first portion 312, thereby providing user input as described earlier.

Some embodiments described herein relate to a computer storage product with a non-transitory computer-readable medium (also referred to as a non-transitory processor-readable medium) having instructions or computer code thereon for performing various computer-implemented operations. The computer-readable medium (or processor-readable medium) is non-transitory in the sense that it does not include transitory propagating signals (e.g., a propagating electromagnetic wave carrying information on a transmission medium such as space or a cable). The media and computer code (also referred to herein as code) may be those designed and constructed for the specific purpose or purposes. Examples of non-transitory computer-readable media include, but are not limited to: flash memory, magnetic storage media such as hard disks, optical storage media such as Compact Disc/Digital Video Discs (CD/DVDs), Compact Disc-Read Only Memories (CD-ROMs), magneto-optical storage media such as optical disks, carrier wave signal processing modules, and hardware devices that are specially configured to store and execute program code, such as Application-Specific Integrated Circuits (ASICs), Programmable Logic Devices (PLDs), Read-Only Memory (ROM) and Random-Access Memory (RAM) devices.

Examples of computer code include, but are not limited to, micro-code or microinstructions, machine instructions, such as produced by a compiler, code used to produce a web service, and files containing higher-level instructions that are executed by a computer using an interpreter. For example, embodiments may be implemented using Java, C++, or other programming languages and/or other development tools.

Where methods and/or schematics described above indicate certain events and/or flow patterns occurring in certain order, the ordering of certain events and/or flow patterns may be modified. Additionally certain events may be performed concurrently in parallel processes when possible, as well as performed sequentially.

## Claims

1. A method, comprising:
receiving, wirelessly from a first device (100), at a second device (160), user input information associated with a user, the first device (100) comprising one or more input sensors or interfaces for receiving input from the user;
receiving, wirelessly at the second device (160) additional information associated with the user, the additional information including information from a third device;
identifying, based on the user input information and the additional information, one or more actions for execution including traversing a structure of a plurality of actions, the structure selected from the group consisting of a directed graph, an undirected graph, a finite state model, a decision tree, and a flowchart; and
transmitting, from the second device, an indication of the one or more actions to be executed to the third device wirelessly.

2. The method of claim 1, further comprising receiving, from the first device (100), at the second device (160), fitness information associated with the user, the identifying the one or more actions based on the user input information, the fitness information, and the additional information, wherein the fitness information is selected from the group consisting of physiological information, geospatial information, timing information, and combinations thereof.

3. The method of claim 1, the user input information selected from the group consisting of tactile entry information, motion information, sensed information, audio information, and combinations thereof.

4. The method of claim 1, the additional information selected from the group consisting of information received from the second device (160), information from a third device, and combinations thereof.

5. The method of claim 1, the receiving the user input information including receiving the user input information from the first device (100) on a periodic basis.

6. The method of claim 1, further comprising executing at least one action of the one or more actions at the third device.

7. A system comprises a first device (100) and a second device (160), the first device (100) comprising one or more input sensors or interfaces for receiving input from the user and the second device (160) comprising:
a communication module (180) configured for:
receiving, wirelessly from the first device (100), user input information associated with a user; and
receiving, wirelessly from a third device, additional information associated with the user; and
an action module (170) configured for identifying, based on the user input information and the additional information, one or more actions by traversing a structure of a plurality of actions for execution, the structure selected from the group consisting of a directed graph, an undirected graph, a finite state model, a decision tree, and a flowchart,
the communication module further configured for:
transmitting an indication of the one or more actions from the second device to the third device wirelessly.

8. The system of claim 7, further comprising receiving, from the first device (100), at the second device (160), fitness information associated with the user, the identifying the one or more actions based on the user input information, the fitness information, and the additional information, wherein the fitness information is selected from the group consisting of physiological information, geospatial information, timing information, and combinations thereof.

9. The system of claim 7, the user input information selected from the group consisting of tactile entry information, motion information, sensed information, audio information, and combinations thereof.

10. The system of claim 7, the communication module (180) configured for receiving fitness information associated with the user when the first device (100) is in physical proximity of the second device (160).

## Patentansprüche

1. Verfahren, umfassend:
Empfangen von Benutzereingabeinformationen, die einem Benutzer zugeordnet sind, drahtlos von einer ersten Vorrichtung (100) an einer zweiten Vorrichtung (160), wobei die erste Vorrichtung (100) einen oder mehrere Eingabesensoren oder Schnittstellen zum Empfangen von Eingaben vom Benutzer umfasst;
Empfangen zusätzlicher Informationen, die dem Benutzer zugeordnet sind, drahtlos an der zweiten Vorrichtung (160), wobei die zusätzlichen Informationen Informationen von einer dritten Vorrichtung beinhalten;
Identifizieren einer oder mehrerer Aktionen zur Ausführung, einschließlich Durchlaufen einer Struktur einer Vielzahl von Aktionen, basierend auf den Benutzereingabeinformationen und den zusätzlichen Informationen, wobei die Struktur ausgewählt ist aus der Gruppe, bestehend aus einem gerichteten Graphen, einem ungerichteten Graphen, einem endlichen Zustandsmodell, einem Entscheidungsbaum und einem Flussdiagramm; und
Übertragen einer Angabe der einen oder der mehreren auszuführenden Aktionen von der zweiten Vorrichtung drahtlos an die dritte Vorrichtung.

2. Verfahren nach Anspruch 1, ferner umfassend das Empfangen von Fitnessinformationen, die dem Benutzer zugeordnet sind, von der ersten Vorrichtung (100) an der zweiten Vorrichtung (160), das Identifizieren der einen oder der mehreren Aktionen basierend auf den Benutzereingabeinformationen, den Fitnessinformationen und den zusätzlichen Informationen, wobei die Fitnessinformationen ausgewählt sind aus der Gruppe, bestehend aus physiologischen Informationen, raumbezogenen Informationen, Zeitinformationen und Kombinationen davon.

3. Verfahren nach Anspruch 1, wobei die Benutzereingabeinformationen ausgewählt sind aus der Gruppe, bestehend aus taktilen Eingabeinformationen, Bewegungsinformationen, erfassten Informationen, Audioinformationen und Kombinationen davon.

4. Verfahren nach Anspruch 1, wobei die zusätzlichen Informationen ausgewählt sind aus der Gruppe bestehend aus Informationen, die von der zweiten Vorrichtung (160) empfangen wurden, Informationen von einer dritten Vorrichtung und Kombinationen davon.

5. Verfahren nach Anspruch 1, wobei das Empfangen der Benutzereingabeinformationen das periodische Empfangen der Benutzereingabeinformationen von der ersten Vorrichtung (100) beinhaltet.

6. Verfahren nach Anspruch 1, ferner umfassend das Ausführen mindestens einer Aktion der einen oder der mehreren Aktionen an der dritten Vorrichtung.

7. System, umfassend eine erste Vorrichtung (100) und eine zweite Vorrichtung (160), wobei die erste Vorrichtung (100) einen oder mehrere Eingangssensoren oder Schnittstellen zum Empfangen von Eingaben von dem Benutzer umfasst und die zweite Vorrichtung (160) Folgendes umfasst:
ein Kommunikationsmodul (180), das zu Folgendem konfiguriert ist:
Empfangen von Benutzereingabeinformationen, die einem Benutzer zugeordnet sind, drahtlos von der ersten Vorrichtung (100); und
Empfangen von zusätzlichen Informationen, die dem Benutzer zugeordnet sind, drahtlos von einer dritten Vorrichtung; und
ein Aktionsmodul (170), das dazu konfiguriert ist, basierend auf den Benutzereingabeinformationen und den zusätzlichen Informationen eine oder mehrere Aktionen durch Durchlaufen einer Struktur einer Vielzahl von zur Ausführung bestimmten Aktionen zu identifizieren, wobei die Struktur ausgewählt ist aus der Gruppe, bestehend aus einem gerichteten Graphen, einem ungerichteten Graphen, einem endlichen Zustandsmodell, einem Entscheidungsbaum und einem Flussdiagramm,
wobei das Kommunikationsmodul ferner zu Folgendem konfiguriert ist:
Übertragen einer Angabe der einen oder der mehreren Aktionen von der zweiten Vorrichtung drahtlos an die dritte Vorrichtung.

8. System nach Anspruch 7, ferner umfassend das Empfangen von Fitnessinformationen, die dem Benutzer zugeordnet sind, von der ersten Vorrichtung (100) an der zweiten Vorrichtung (160), das Identifizieren der einen oder der mehreren Aktionen basierend auf den Benutzereingabeinformationen, den Fitnessinformationen und den zusätzlichen Informationen, wobei die Fitnessinformationen ausgewählt sind aus der Gruppe, bestehend aus physiologischen Informationen, raumbezogenen Informationen, Zeitinformationen und Kombinationen davon.

9. System nach Anspruch 7, wobei die Benutzereingabeinformationen ausgewählt sind aus der Gruppe, bestehend aus taktilen Eingabeinformationen, Bewegungsinformationen, erfassten Informationen, Audioinformationen und Kombinationen davon.

10. System nach Anspruch 7, wobei das Kommunikationsmodul (180) dazu konfiguriert ist, Fitnessinformationen zu empfangen, die dem Benutzer zugeordnet sind, wenn sich die erste Vorrichtung (100) in physischer Nähe der zweiten Vorrichtung (160) befindet.

## Revendications

1. Procédé, comprenant :
la réception, de manière sans fil provenant d'un premier dispositif (100), au niveau d'un deuxième dispositif (160), d'informations d'entrée d'utilisateur associées à un utilisateur, le premier dispositif (100) comprenant un ou plusieurs capteurs ou interfaces d'entrée pour recevoir une entrée provenant de l'utilisateur ;
la réception, de manière sans fil au niveau du deuxième dispositif (160), d'informations supplémentaires associées à l'utilisateur, les informations supplémentaires comportant des informations provenant d'un troisième dispositif ;
l'identification, sur la base des informations d'entrée d'utilisateur et des informations supplémentaires, d'une ou plusieurs actions à exécuter comportant le fait de traverser une structure d'une pluralité d'actions, la structure étant sélectionnée dans le groupe constitué d'un graphe orienté, d'un graphe non orienté, d'un modèle d'état fini, d'un arbre de décision et d'un organigramme ; et
la transmission de manière sans fil, provenant du deuxième dispositif, d'une indication des une ou plusieurs actions à exécuter au troisième dispositif.

2. Procédé selon la revendication 1, comprenant également la réception, provenant du premier dispositif (100), au niveau du deuxième dispositif (160), d'informations de forme physique associées à l'utilisateur, l'identification des une ou plusieurs actions sur la base des informations d'entrée d'utilisateur, des informations de forme physique, et des informations supplémentaires, dans lequel les informations de forme physique sont sélectionnées dans le groupe constitué d'informations physiologiques, d'informations géospatiales, d'informations de synchronisation, et de combinaisons de celles-ci.

3. Procédé selon la revendication 1, les informations d'entrée utilisateur étant sélectionnées dans le groupe constitué d'informations d'entrée tactile, d'informations de mouvement, d'informations détectées, d'informations audio, et des combinaisons de celles-ci.

4. Procédé selon la revendication 1, les informations supplémentaires étant sélectionnées dans le groupe constitué d'informations reçues provenant du deuxième dispositif (160), d'informations provenant d'un troisième dispositif et de combinaisons de celles-ci.

5. Procédé selon la revendication 1, la réception des informations d'entrée d'utilisateur comportant la réception des informations d'entrée d'utilisateur provenant du premier dispositif (100) sur une base périodique.

6. Procédé selon la revendication 1, comprenant également l'exécution d'au moins une action parmi les une ou plusieurs actions au niveau du troisième dispositif.

7. Système qui comprend un premier dispositif (100) et un deuxième dispositif (160), le premier dispositif (100) comprenant un ou plusieurs capteurs ou interfaces d'entrée destinés à recevoir les entrées provenant de l'utilisateur et le deuxième dispositif (160) comprenant :
un module de communication (180) configuré pour :
la réception, de manière sans fil provenant du premier dispositif (100), d'informations d'entrée d'utilisateur associées à un utilisateur ; et
la réception, de manière sans fil provenant d'un troisième dispositif, d'informations supplémentaires associées à l'utilisateur ; et
un module d'action (170) configuré pour l'identification, sur la base des informations d'entrée d'utilisateur et des informations supplémentaires, d'une ou plusieurs actions en traversant une structure d'une pluralité d'actions destinées à être exécutées, la structure étant sélectionnée dans le groupe constitué d'un graphe orienté, d'un graphe non orienté, d'un modèle d'état fini, d'un arbre de décision, et d'un organigramme, le module de communication étant également configuré pour :
la transmission de manière sans fil d'une indication des une ou plusieurs actions du deuxième dispositif au troisième dispositif.

8. Système selon la revendication 7, comprenant également la réception, provenant du premier dispositif (100), au niveau du deuxième dispositif (160), d'informations de forme physique associées à l'utilisateur, l'identification des une ou plusieurs actions sur la base des informations d'entrée d'utilisateur, des informations de forme physique, et des informations supplémentaires, dans lequel les informations de forme physique sont sélectionnées dans le groupe constitué d'informations physiologiques, d'informations géospatiales, d'informations de synchronisation, et des combinaisons de celles-ci.

9. Système selon la revendication 7, les informations d'entrée utilisateur étant sélectionnées dans le groupe constitué d'informations d'entrée tactile, d'informations de mouvement, d'informations détectées, d'informations audio, et des combinaisons de celles-ci.

10. Système selon la revendication 7, le module de communication (180) étant configuré pour recevoir des informations de forme physique associées à l'utilisateur lorsque le premier dispositif (100) est à proximité physique du deuxième dispositif (160).
